# EUROPEAN PATENT SPECIFICATION

(11) **EP 2 962 701 B1**
(45) Date of publication and mention of the grant of the patent: **22.01.2020**
(21) Application number: 15175274.8
(22) Date of filing: 03.07.2015
(51) Int. Cl.: A61L 9/12, A01M 1/20, B60H 3/00

(54) **PIERCEABLE CARRYING DEVICE FOR A VOLATILE MATERIAL**
DURCHBOHRBARE TRÄGERVORRICHTUNG FÜR EINEN FLÜCHTIGEN STOFF
DISPOSITIF PERFORABLE POUR LE TRANSPORT D'UN PRODUIT VOLATIL

(30) Priority: 04.07.2014 TW 103211950 U
(43) Date of publication of application: 06.01.2016
(73) Proprietor: Aromate Industries Co., Ltd., New Taipei City 231 (TW)
(72) Inventor: WU, Henry, New Taipei City (TW)
(74) Representative: Viering, Jentschura & Partner mbB Patent- und Rechtsanwälte

(56) References cited:
- EP-A1- 0 214 918
- EP-A1- 1 500 357
- EP-A1- 2 419 148
- JP-A- 2003 144 032

## Description

### BACKGROUND OF THE INVENTION

### 1. Field of the Invention

The instant disclosure relates to an easy-to-break carrying device for volatiles; in particular, to an easy-to-break carrying device for volatiles for receiving a volatile such as a car air freshener, car deodorant, indoor air freshener, indoor deodorant, or pesticide.

### 2. Description of Related Art

Traditionally, a volatile such as car air freshener, car deodorant, indoor air freshener, indoor deodorant, or pesticide is received in a packaging container, and the packaging container has an opening, and a sealing film is disposed on the opening to avoid the volatile received in the packaging container volatilizing, so as to facilitate storage and transport. When the consumer would like to use utilize the volatile, the sealing film of the opening is removed by the consumer, and the volatile received in the packaging container volatilizes into the air.

Since such traditional carrying devices for volatiles having a sealing film which needs to be removed in a manual way are inconvenient for consumers. In addition, after the sealing film is removed, the opening of the packaging container is fully opened, and the volatilization rate of the volatile is uncontrollable, such that the volatile is liable to overflow out of the packaging container to pollute the surroundings.

In traditional carrying devices for volatiles, some packaging containers have a destruction assembly disposed thereon, the destruction assembly has a bendable destruction component, and the destruction component has a plurality of piercing portions to pierce the sealing film when the user would like to press the destruction component to break the sealing film of the carrying device, so that the volatile can spread out from the sealing film having holes.

However, the piercing portion on the destruction assembly of the carrying device for the volatile in the prior art is disposed perpendicular to the surface of the sealing film, so if the destruction component is collided into or extruded by an external force, the piercing portion will break the sealing film, and the sealing film will lose its sealing function.

Due to the abovementioned reasons, the carrying device for the volatile in the prior art has many disadvantages, thus, to develop an improved carrying device for volatiles to overcome the abovementioned drawbacks is important in the industry.

EP 2 419 148 A1 discloses an apparatus for delivering a volatile material in a continuous manner. The apparatus includes a delivery engine having a reservoir for containing a volatile material; a rupturable substrate secured to the reservoir; a rupture element positioned adjacent to the rupturable substrate; and a breathable membrane enclosing the reservoir, rupturable substrate and rupture element.

### SUMMARY OF THE INVENTION

The object of the invention is achieved by the subject matter of claim 1. Advantageous embodiment are disclosed in the dependent claims.

An example of the instant disclosure provides an easy-to-break carrying device for volatiles, which is adapted for a car air freshener or deodorant. It can remain sealed during transportation and storage, avoiding the volatile spreading out. The user can easily utilize a destruction assembly of the easy-to-break carrying device for volatiles, to break a sealing member of the carrying device when the user would like to use the carrying device, and the volatile stored in the carrying device can spread into the air.

The easy-to-break carrying device for the volatile in the instant disclosure includes a packaging case, a sealing film, a destruction assembly, and a permeable film. The packaging case has a first accommodating space for receiving the volatile and a second accommodating space connecting to the first accommodating space having an opening. The sealing film covers the opening of the first accommodating space. The destruction assembly is disposed in the second accommodating space and located above the sealing film. The destruction assembly has a housing body, two extending plates, and a plurality of piercing portions. One end of the two extending plates connects to the corresponding two sides of the housing body having an inside edge, and another end of the two extending plates cooperatively extends towards the housing body having a central position. The plurality of piercing portions are disposed at the at least one side of the two extending plates, and the plurality of piercing portions are protrudingly disposed at the at least one side of the extending plate. The permeable film is disposed at the opening of the second accommodating space of the housing body and covers it above the destruction assembly. The permeable film is formed with clouds of tiny pores.

The two extending plates of the destruction assembly have a bendable elasticity, and the plurality of piercing portions are displaced downward along the extending plate when the two extending plates are depressed and curved downward, such that the sealing film is broken by the plurality of piercing portions.

In one example of the instant disclosure, the plurality of piercing portions are disposed on a side of one end of the two extending plates close to each other.

In another example of the instant disclosure, the plurality of piercing portions are disposed at two sides of the two extending plates.

In a further example of the instant disclosure, each of the extending plates is close to two sides of one end of another extending plate, and respectively has a connecting portion connecting to two sides of terminal ends of the another extending plate.

In the further example of the instant disclosure, a protrusion protruded from a top surface of the extending plate is respectively disposed at each of top surfaces of each of the two extending plates.

In order to further appreciate the characteristics and technical contents of the present invention, references are hereunder made to the detailed descriptions and appended drawings in connection with the present invention. However, the appended drawings are merely shown for exemplary purposes, rather than being used to restrict the scope of the present invention.

### BRIEF DESCRIPTION OF THE DRAWINGS

FIG 1 shows a perspective view of a first embodiment of the instant disclosure;
FIG 2 shows an exploded perspective view of the first embodiment of the instant disclosure;
FIG 3 shows a combined cross-sectional view of the first embodiment of the instant disclosure;
FIG 4 shows a perspective view of a destruction assembly used in the first embodiment of the instant disclosure;
FIG 5 shows a perspective view from another side of the destruction assembly used in the first embodiment of the instant disclosure;
FIG 6 shows an enlarged cross-sectional view of the destruction assembly used in the first embodiment of the instant disclosure; and
FIG 7 shows a perspective view of the destruction assembly used in a second embodiment of the instant disclosure.

### DETAILED DESCRIPTION OF THE PREFERRED EMBODIMENTS

### <First Embodiment>

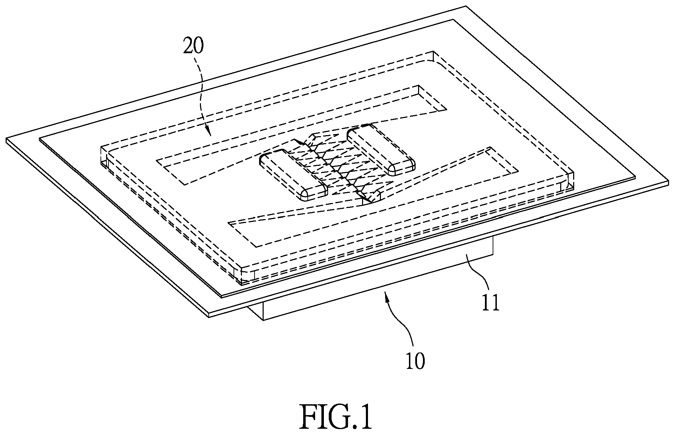

Please refer to FIGs 1 to 3. An easy-to-break carrying device for a volatile of a first embodiment of the instant disclosure includes a packaging case 10, a sealing film 13, a destruction assembly 20, and a permeable film 14.

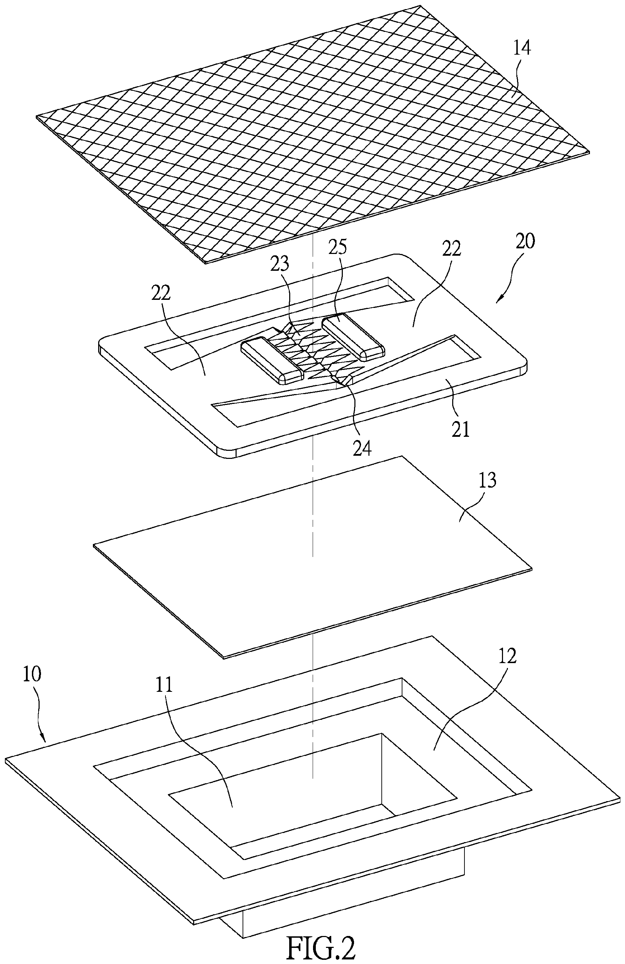

The packaging case 10 is a container formed by injection molding in a plastic material, or formed by pressing in the plastic material, which has a first accommodating space 11 having a closed bottom and an open top, and a second accommodating space 12 connecting to the opening of the first accommodating space 11 and having a width which is larger than the first accommodating space 11 having a width. As shown in FIG 2, the sealing film 13 covers the opening of the first accommodating space 11, and completely seals the opening of the first accommodating space 11 to form an enclosed space, so as to receive a volatile 30 therein.

Examples of the volatile 30 include, but are not limited to an aromatic, deodorant, insecticide, desiccant, and camphor. The volatile 30 can be stored long-term in the first accommodating space 11 and avoid volatilization via sealing by the sealing film 13. In the embodiment, examples of the sealing film 13 include, but are not limited to a foil, and film. The material having tightness which can resist erosion by the volatile 30, can be selected to be the sealing film 13.

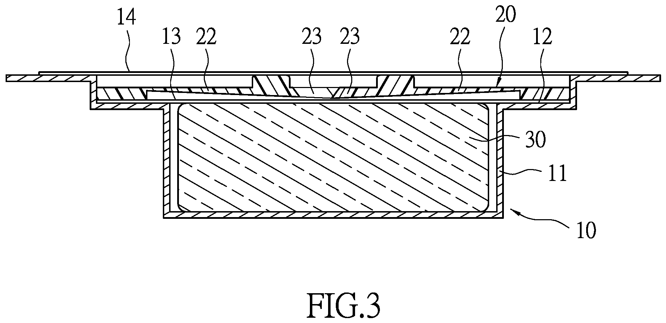

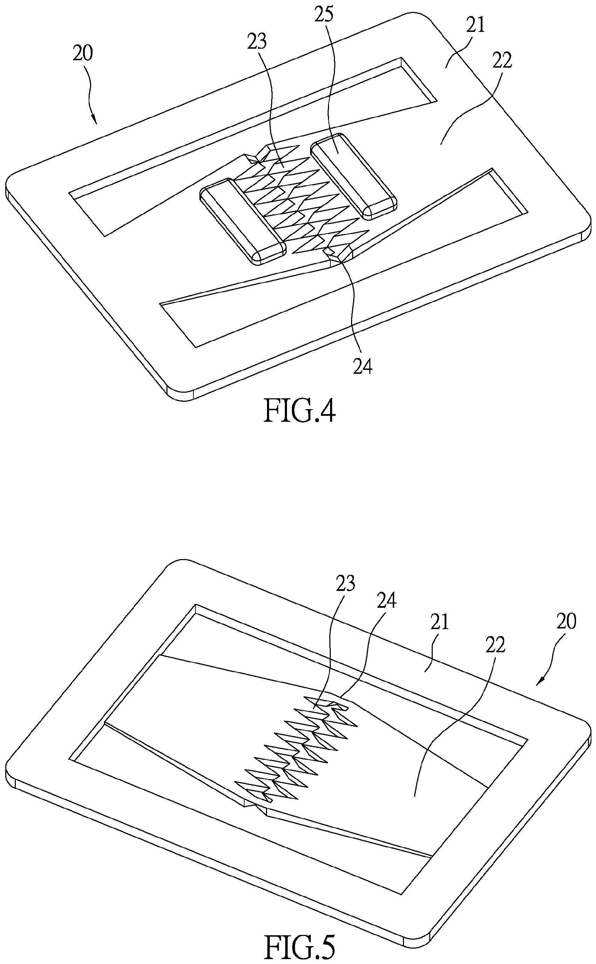

The destruction assembly 20 is received in the second accommodating space 12 of the packaging case 10, and is abutted on the sealing film 13 having a top surface. The permeable film 14 is above and covers the opening of the second accommodating space 12 to enshroud it, and the destruction assembly 20 is hidden beneath the permeable film 14. The permeable film 14 is made of a material having a porosity such as a non-woven material, or made of a sheet-like material such as a foil or a film formed with clouds of tiny pores thereon. The permeable film 14 has air permeability, and can help the volatile 30 to diffuse via a capillary action. In the embodiment of the instant disclosure, the permeable film 14 and the sealing film 13 are disposed very close to each other, when the sealing film 13 is broken to allow the volatile 30 spreading out through the sealing film 13, part of the volatile 30 contacts with the permeable film 14. Since the permeable film 14 is formed with tiny pores, these can generate the capillary action to facilitate diffusion of the volatile 30. The destruction assembly 20 can pierce the sealing film 13 and form pores for the volatile 30 to contact with outside air. As shown in FIGs 3 and 4, the destruction assembly 20 of the first embodiment of the instant disclosure is formed in one piece of plastic material using injection molding, has a housing body 21, two extending plates 22, and a plurality of piercing portions 23, wherein the housing body 21 is a hollow frame, and its size coordinately corresponds to the second accommodating space 12, so as to be received in the second accommodating space 12. One end of the two extending plates 22 connects to corresponding two sides of the housing body 21 having an inside edge, and another end of the two extending plates 22 cooperatively extends toward the housing body 21 having a central position. In the embodiment, the extending plate 22 and the housing body 21 are formed in one piece of plastic material by injection molding, such that the extending plate 22 has a bendable elasticity. A plurality of piercing portions 23 are disposed at the two extending plates 22 having one side of one end where the two extending plates 22 are close to each other, and each of the piercing portions 23 is protrudingly disposed at a side edge of the extending plate 22 and is in parallel with the extending plate 22 having a bottom surface. Each of the piercing portions 23 respectively has a sharp terminal end, as shown in FIGs 2 and 5, when the extending plate 22 is depressed and curved downward, each of the piercing portions 23 is displaced downward along the extending plate 22, and each of the piercing portions 23 having sharp terminal ends pierces into the sealing film 13, such that the sealing film 13 is broken to form a plurality of pores.

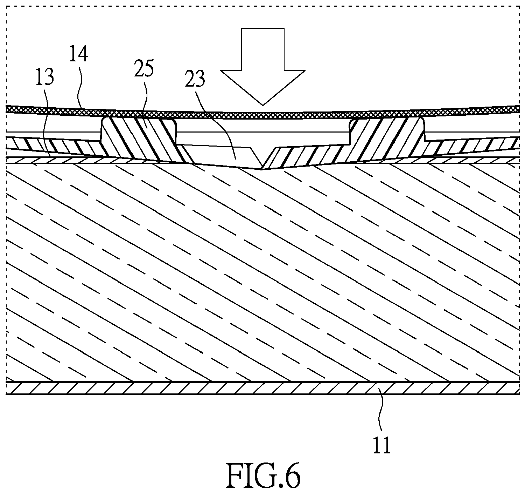

As shown in FIG 6, due to the sharp terminal end of the piercing portion 23 in the instant disclosure having a protruding direction in parallel with a surface of the sealing film 13, when the piercing portion 23 is displaced downward by a movement of the extending plate 22, each of the piercing portions 23 pierces deeply into the sealing film 13. The depth has to be larger than the sealing film 13 having a deformation endurance limit, so as to break the sealing film 13.

As shown in FIGs 3 and 6, when the easy-to-break carrying device for the volatile in the instant disclosure is used, the user can press the two extending plates 22 of the destruction assembly 20, and the piercing portions 23 disposed on the extending plate 22 pierce into the sealing film 13 to form a plurality of pores, such that the volatile 30 received in the first accommodating space 11 can contact with the outside air through these pores. One of the main features of the destruction assembly 20 in the instant disclosure is that the piercing portions 23 are disposed at the side of the extending plate 22 in a horizontally extending manner, such that each of the piercing portions 23 has an extending direction in parallel with the extending plate 22 having a bottom surface, and is in parallel with the sealing film 13. In such a way, when the extending plate 22 of the destruction assembly 20 is accidentally collided into, the piercing portions 23 are not liable to break the sealing film 13, keeping the function of the sealing film 13.

Moreover, as shown in FIGs 3, 4, and 5, two sides of the one end where the two extending plates 22 are close to each other in the instant disclosure respectively have a connecting portion 24 connecting to two sides of the terminal ends of the two extending plates 22, so as to connect the terminal ends together where the two extending plates 22 are close to each other. The two connecting portions 24 have a tapered central thickness, and a central section of the two connecting portions 24 is easily bent. In the instant disclosure, the purpose of the connecting portion 24 being disposed at the two sides of the end where the two extending plates 22 are close to each other is that the bending of the terminal end of the two extending plates 22 is limited, and the terminal end of the two extending plates 22 is not curved downward arbitrarily, so as to avoid the two extending plates 22 of the destruction assembly 20 undergoing downward bending deformation by slight touching, when the easy-to-break carrying device for the volatile in the instant disclosure is stored and transported, such that the sealing film 13 is broken by the piercing portions 23 of the extending plate 22.

In addition, as shown in FIG 3, a protrusion 25 is further disposed at the top surface of the extending plate 22, so that the user can easily press the top surface of the extending plate 22.

### <Second Embodiment>

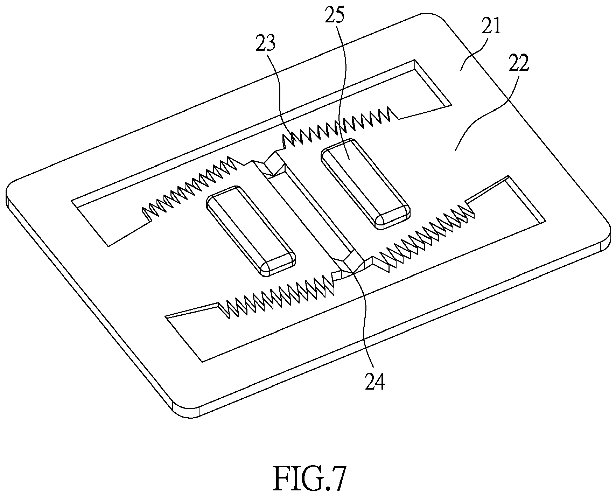

As shown in FIG 7, a difference between the destruction assembly 20 of the second embodiment and that of the first embodiment is that the piercing portions 23 of the second embodiment are disposed at the two sides of the terminal end close to the two extending plates 22. In the embodiment, the piercing portions 23 are disposed at a different position, such that the sealing film 13 is pierced and broken at a different position by the piercing portions 23.

In the embodiment, although the piercing portions 23 are disposed at a different position, the piercing portions 23 also can break the sealing film 13 when the extending plate 22 is bent downward, and the volatile 30 received in the first accommodating space 11 can spread out from the sealing film 13 through the pierced pores.

### <Efficacy of Embodiments>

In summary, the easy-to-break carrying device for volatiles of the instant disclosure has an advantage that the piercing portions 23 disposed on the destruction assembly 20 having the extending direction in the instant disclosure are in parallel with the extending plate 22 having a bottom surface. Therefore, each of the piercing portions 23 having a protruding direction with a sharp terminal end is in parallel with the sealing film 13 having a surface. Hence, during transportation and storage of the easy-to-break carrying device for volatiles of the instant disclosure, the piercing portions 23 are not liable to break the sealing film 13 when the two extending plates 22 of the destruction assembly 20 are extruded and bent toward the sealing film 13, and this can reduce the chances of the sealing film 13 being broken due to collision or extrusion by accident, so as to enhance the reliability of the easy-to-break carrying device for volatiles in the instant disclosure.

The descriptions illustrated *supra* set forth simply the preferred embodiments of the present invention; however, the characteristics of the present invention are by no means restricted thereto. All changes, alterations, or modifications conveniently considered by those skilled in the art are deemed to be encompassed within the scope of the present invention delineated by the following claims.

## Claims

1. A pierceable carrying device for a volatile (30), comprising:
a packaging case (10) which has a first accommodating space (11) for receiving the volatile (30) and a second accommodating space (12) connecting to the first accommodating space (11); the first accommodating space (11) having an opening, and a width of the second accommodating space (12) is larger than a width of the first accommodating space (11);
a sealing film (13) which covers the opening of the first accommodating space (11);
a destruction assembly (20) which is disposed in the second accommodating space (12), wherein the destruction assembly (20) has a housing body (21), two extending plates (22), and a plurality of piercing portions (23), the housing body (21) which is received in the second accommodating space (12) is a hollow frame abutted on the sealing film (13), wherein the housing body (21) has two opposite inside edges, one end of the two extending plates (22) connected to the two opposite inside edges of the housing body (21) respectively, another end of the two extending plates (22) has a terminal end, the two terminal ends are cooperatively extending to each other toward a central of the housing body(21);
the plurality of piercing portions (23) are protrudingly disposed at the at least one side of the two extending plates (22), each of the extending plates (22) has a bottom surface facing the sealing film (13), and the plurality of piercing portions (23) are parallel with the bottom surface of the extending plate (22);
two connecting portions (24) disposed between the two terminal ends of the two extending plates (22), the two connecting portions (24) connected to two sides of the two terminal ends of the two extending plates (22) respectively, so that the two extending plates (22) are connected to each other by the two connecting portions (24), each of the connecting portions (24) has a tapered central thickness, wherein a central section of the two connecting portions (24) is bendable, the thickness of the central section is less than the thickness of the two extending plates (22); and
an air-permeable film (14) disposed at the opening of the second accommodating space (12) of the housing body (21) and covers the destruction assembly (20);
wherein the two extending plates (22) of the destruction assembly (20) have a bendable elasticity, and the plurality of piercing portions (23) are displaced downward along the extending plate (22) when the two extending plates (22) are depressed and curved to the sealing film (13), such that the sealing film (13) is broken by the plurality of piercing portions (23).

2. The pierceable carrying device for a volatile (30) as claimed in claim 1, wherein the plurality of piercing portions (23) are disposed on a side of one end of the two extending plates (22) adjacent to each other.

3. The pierceable carrying device for a volatile (30) as claimed in claim 1, wherein the plurality of piercing portions (23) are disposed at two sides of the two extending plates (22).

4. The pierceable carrying device for a volatile (30) as claimed in claim 1, wherein each of the extending plates (22) has a top surface which is opposite to the sealing film (13), a protrusion (25) protruded from the top surface of the extending plate (22) is respectively disposed at each of the top surfaces of each of the two extending plates (22).

5. The pierceable carrying device for a volatile (30) as claimed in claim 4, wherein the destruction assembly (20) is formed in one piece in plastic material.

6. The pierceable carrying device for the volatile (30) as claimed in claim 5, wherein the sealing film (13) is a foil.

## Patentansprüche

1. Durchbohrbare Tragevorrichtung für einen flüchtigen Stoff (30), umfassend:
ein Verpackungsgehäuse (10), das einen ersten Aufnahmeraum (11) zum Aufnehmen des flüchtigen Stoffs (30) und einen zweiten Aufnahmeraum (12) aufweist, der mit dem ersten Aufnahmeraum (11) verbunden ist, wobei der erste Aufnahmeraum (11) eine Öffnung aufweist und eine Breite des zweiten Aufnahmeraums (12) größer als eine Breite des ersten Aufnahmeraums (11) ist,
einen Dichtungsfilm (13), der die Öffnung des ersten Aufnahmeraums (11) bedeckt,
eine Zerstörungsanordnung (20), die in dem zweiten Aufnahmeraum (12) angeordnet ist, wobei die Zerstörungsanordnung (20) einen Gehäusekörper (21), zwei Verlängerungsplatten (22) und eine Mehrzahl von Durchbohrabschnitten (23) aufweist, wobei der Gehäusekörper (21), der in dem zweiten Aufnahmeraum (12) aufgenommen ist, ein hohler Rahmen ist, der an den Dichtungsfilm (13) anstößt, wobei der Gehäusekörper (21) zwei entgegengesetzte Innenkanten aufweist, wobei ein Ende der beiden Verlängerungsplatten (22) mit den beiden entgegengesetzten Innenkanten des Gehäusekörpers (21) verbunden ist, ein anderes Ende der beiden Verlängerungsplatten (22) ein Abschlussende aufweist, die beiden Abschlussenden sich zusammenwirkend zueinander in Richtung zu einer Mitte des Gehäusekörpers (21) erstrecken, die Mehrzahl von Durchbohrabschnitten (23) an der mindestens einen Seite der beiden Verlängerungsplatten (22) vorstehend angeordnet ist, jede der Verlängerungsplatten (22) eine dem Dichtungsfilm (13) zugewandte untere Fläche aufweist und die Mehrzahl von Durchbohrabschnitten (23) parallel zu der unteren Fläche der Verlängerungsplatte (22) ist,
zwei Verbindungsabschnitte (24), die zwischen den beiden Abschlussenden der beiden Verlängerungsplatten (22) angeordnet sind, wobei die beiden Verbindungsabschnitte (24) mit zwei Seiten der beiden Abschlussenden der beiden Verlängerungsplatten (22) verbunden sind, so dass die beiden Verlängerungsplatten (22) durch die beiden Verbindungsabschnitte (24) miteinander verbunden sind, wobei jeder der Verbindungsabschnitte (24) eine verjüngte zentrale Dicke aufweist, wobei ein zentraler Abschnitt der beiden Verbindungsabschnitte (24) biegbar ist, wobei die Dicke des zentralen Abschnitts kleiner als die Dicke der beiden Verlängerungsplatten (22) ist, und
einen luftdurchlässigen Film (14), der an der Öffnung des zweiten Aufnahmeraums (12) des Gehäusekörpers (21) angeordnet ist und die Zerstörungsanordnung (20) bedeckt, wobei die beiden Verlängerungsplatten (22) der Zerstörungsanordnung (20) eine biegbare Elastizität aufweisen und die Mehrzahl von Durchbohrabschnitten (23) entlang der Verlängerungsplatte (22) nach unten verlagert wird, wenn die beiden Verlängerungsplatten (22) niedergedrückt und zum Dichtungsfilm (13) gekrümmt wird, so dass der Dichtungsfilm (13) durch die Mehrzahl von Durchbohrabschnitten (23) durchbrochen wird.

2. Durchbohrbare Tragevorrichtung für einen flüchtigen Stoff (30) nach Anspruch 1, wobei die Mehrzahl von Durchbohrabschnitten (23) auf einer Seite von einem Ende der beiden Verlängerungsplatten (22) benachbart zueinander angeordnet ist.

3. Durchbohrbare Tragevorrichtung für einen flüchtigen Stoff (30) nach Anspruch 1, wobei die Mehrzahl von Durchbohrabschnitten (23) an zwei Seiten der beiden Verlängerungsplatten (22) angeordnet ist.

4. Durchbohrbare Tragevorrichtung für einen flüchtigen Stoff (30) nach Anspruch 1, wobei jede der Verlängerungsplatten (22) eine zu dem Dichtungsfilm (13) entgegengesetzte obere Fläche aufweist, wobei jeweils ein von der oberen Fläche der Verlängerungsplatte (22) hervorstehender Vorsprung (25) an jeder der oberen Flächen von jeder der beiden Verlängerungsplatten (22) angeordnet ist.

5. Durchbohrbare Tragevorrichtung für einen flüchtigen Stoff (30) nach Anspruch 4, wobei die Zerstörungsanordnung (20) einstückig aus Kunststoff gebildet ist,

6. Durchbohrbare Tragevorrichtung für den flüchtigen Stoff (30) nach Anspruch 5, wobei der Dichtungsfilm (13) eine Folie ist.

## Revendications

1. Dispositif de transport perforable pour un produit volatil (30) comprenant :
une boîte d'emballage (10) qui a un premier espace de logement (11) pour recevoir le produit volatil (30) et un second espace de logement (12) se raccordant au premier espace de logement (11) ; le premier espace de logement (11) ayant une ouverture, et une largeur du second espace de logement (12) est supérieure à une largeur du premier espace de logement (11) ;
un film d'étanchéité (13) qui recouvre l'ouverture du premier espace de logement (11) ;
un ensemble de destruction (20) qui est disposé dans le second espace de logement (12), dans lequel l'ensemble de destruction (20) a un corps de boîtier (21), deux plaques d'extension (22) et une pluralité de parties de perforation (23), le corps de boîtier (21) qui est reçu dans le second espace de logement (12) est un bâti creux en butée sur le film d'étanchéité (13), dans lequel le corps de boîtier (21) a deux bords intérieurs opposés, une extrémité des deux plaques d'extension (22) étant raccordée aux deux bords intérieurs opposés du corps de boîtier (21) respectivement, une autre extrémité des deux plaques d'extension (22) a une extrémité de borne, les deux extrémités de borne s'étendent par coopération l'une vers l'autre vers une partie centrale du corps de boîtier (21) ; la pluralité de parties de perforation (23) sont disposées, en saillie, au niveau d'au moins un côté des deux plaques d'extension (22), chacune des plaques d'extension (22) a une surface inférieure faisant face au film d'étanchéité (13), et la pluralité de parties de perforation (23) sont parallèles à la surface inférieure de la plaque d'extension (22) ;
deux parties de raccordement (24) disposées entre les deux extrémités de borne des deux plaques d'extension (22), les deux parties de raccordement (24) étant raccordées aux deux côtés des deux extrémités de borne des deux plaques d'extension (22) respectivement, de sorte que les deux plaques d'extension (22) sont raccordées entre elles par les deux parties de raccordement (24), chacune des parties de raccordement (24) a une épaisseur centrale progressivement rétrécie, dans lequel une section centrale des deux parties de raccordement (24) peut être pliée, l'épaisseur de la section centrale est inférieure à l'épaisseur des deux plaques d'extension (22) ; et
un film perméable à l'air (14) disposé au niveau de l'ouverture du second espace de logement (12) du corps de boîtier (21) et recouvre l'ensemble de destruction (20) ;
dans lequel les deux plaques d'extension (22) de l'ensemble de destruction (20) ont une élasticité pliable, et la pluralité de parties de perforation (23) sont déplacées vers le bas le long de la plaque d'extension (22) lorsque les deux plaques d'extension (22) sont enfoncées et incurvées par rapport au film d'étanchéité (13), de sorte que le film d'étanchéité (13) est cassé par la pluralité de parties de perforation (23).

2. Dispositif de transport perforable pour un produit volatil (30) selon la revendication 1, dans lequel la pluralité de parties de perforation (23) sont disposées sur un côté d'une extrémité des deux plaques d'extension (22) adjacentes entre elles.

3. Dispositif de transport perforable pour un produit volatil (30) selon la revendication 1, dans lequel la pluralité de parties de perforation (23) sont disposées au niveau des deux côtés des deux plaques d'extension (22).

4. Dispositif de transport perforable pour un produit volatil (30) selon la revendication 1, dans lequel chacune des plaques d'extension (22) a une surface supérieure qui est opposée au film d'étanchéité (13), une saillie (25) faisant saillie de la surface supérieure de la plaque d'extension (22) est respectivement disposée au niveau de chacune des surfaces supérieures de chacune des deux plaques d'extension (22).

5. Dispositif de transport perforable pour un produit volatil (30) selon la revendication 4, dans lequel l'ensemble de destruction (20) est formé avec une pièce de matière plastique.

6. Dispositif de transport perforable pour un produit volatil (30) selon la revendication 5, dans lequel le film d'étanchéité (13) est une feuille.
